# EUROPEAN PATENT APPLICATION

(11) **EP 1 958 934 A1**
(43) Date of publication of application: **20.08.2008**
(21) Application number: 07090026.1
(22) Date of filing: 16.02.2007
(51) Int. Cl.: C07D 207/34, C07D 231/40, C07D 233/90, C07D 249/06, C07D 401/04, A61K 31/415, A61K 31/4192, A61K 31/4164, A61P 29/00

(54) **Tetrahydronaphthalenylamides, a process for their production and their use as anti-inflammatory agents**

(71) Applicant: Bayer Schering Pharma Aktiengesellschaft, 13353 Berlin (DE); AstraZeneca AB, 151 85 Södertälje (SE)
(72) Inventor: Berger, Markus, 13347 Berlin (DE); Rehwinkel, Hartmut, 10961 Berlin (DE); Schäcke, Heike, 10115 Berlin (DE); May, Ekkehard, 13465 Berlin (DE); Skuballa, Werner, 13465 Berlin (DE); Künzer, Hermann, 13347 Berlin (DE)

(57) **Abstract**

The present invention relates to the compounds of formula I, 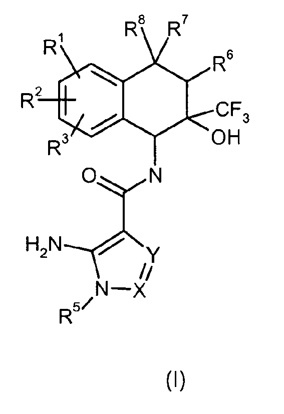 processes for their production and their use as anti-inflammatory agents.

## Description

The present invention relates to compounds of formula I, a process for their production and their use as anti-inflammatory agents.

From the prior art of DE 100 38 639 and WO 02/10143, anti-inflammatory agents of the following general formula 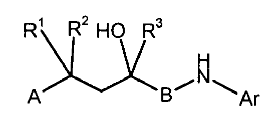 are known, in which the Ar radical comprises phthalides, thiophthalides, benzoxazinones or phthalazinones. In the experiment, these compounds show dissociations of action between anti-inflammatory and undesirable metabolic actions and are superior to the previously described nonsteroidal glucocorticoids or exhibit at least just as good an action. Nevertheless these compounds possess a linear structure as also the compounds disclosed in W02006/108699, and W02007/000334 whereas the compounds disclosed in the following publications do have a bicyclic ring moiety:
WO2005/034939, WO2006/100100, WO2006/108712, .

However, the selectivity of the compounds of the prior art towards the glucocorticoid receptor (GR) compared to the other steroid receptors still requires improvement. Also dissociation between wanted anti inflammatory effects and unwanted metabolic side effects requires improvement.

It was therefore the object of this invention to make compounds available whose selectivity towards the glucocorticoid receptor (GR) is improved compared to the other steroid receptors and which show a better dissociation regarding potential side effects or at least the same level of effect.
This object is achieved by the compounds according to the claims.

This invention therefore relates to stereoisomers of general formula I 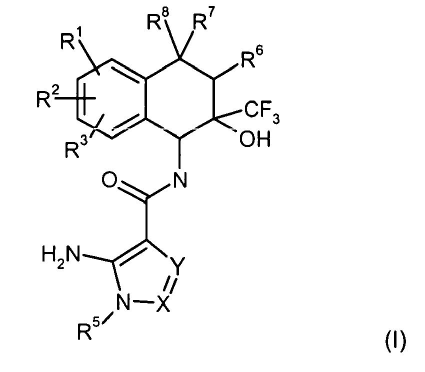 in which
- X: is N or C-R⁴
- Y: is N or C-R⁴
- R¹, R² R³: independently of one another, are selected from the group consisting of hydrogen, halogen, cyano, nitro, hydroxy, or (C₁-C₅)-alkyl, (C₁-C₅)-halo-alkyl, (C₁-C₅)alkoxy, (C₁-C₅)halo-alkoxy and COOR⁹,
and in which two vicinal substituents together may form a group that is selected from the groups
-O-(CH₂)ₚ-O-, -O-(CH₂)ₚ-CH₂-, -O-CH=CH-, -(CH₂)ₚ₊₂-, -NH-(CH₂)ₚ₊₁-,
-N(C₁-C₃-alkyl)-(CH₂)ₚ₊₁-, and -NH-N=CH-,
in which p = 1 or 2, and in which R⁹ means hydrogen or C₁-C₄-alkyl
- R⁴: is selected from the group consisting of hydrogen, (C₁-C₅)-alkyl, or (C₁-C₅)-halo-alkyl, and should two R⁴ moities be present in one molecule these have independent meanings,
- R⁵: means a phenyl, pyridinyl or pyrimidinyl rest
which may have 1-3 substituents independently selected from the group consisting of halogen, cyano, nitro hydroxy, or (C₁-C₅)-alkyl, (C₁-C₅)-halo-alkyl, (C₁-C₅)alkoxy, (C₁-C₅)halo-alkoxy and COOR⁹, in which R⁹ has the above identified meaning,
and in which two vicinal substituents together may form a group that is selected from the groups
-O-(CH₂)ₚ-O-, -O-(CH₂)ₚ-CH₂-, -O-CH=CH-, -(CH₂)_{P+2}-, -NH-(CH₂)ₚ₊₁-,
-N(C₁-C₃-alkyl)-(CH₂)ₚ₊₁-, and -NH-N=CH-,
- R⁶: is selected from the group consisting of hydrogen or (C₁-C₅)-alkyl or (C₁-C₅)-halo-alkyl
- R⁷, R⁸: independently of one another, are selected from the group consisting of hydrogen or (C₁-C₅)-alkyl, (C₁-C₅)-halo-alkyl
or in which R⁶ and R⁷ together or R⁶ and R⁸ together or R⁷ and R⁸ together may form a C₃-C₈ cycloalkyl ring.

If R⁵ is a pyrimidinyl or a pyridinyl group this group may be bound by any position to the pyrrole, imidazole, triazole or pyrazole ring.

Unless otherwise notifed the term "alkyl" refers to straight or branched derivatives. For example, the term propyl comprises *ⁿ*-propyl and *^{iso}*-propyl, the term butyl comrises *ⁿ*-butyl, *^{iso}*-butyl and *^{tert}*-butyl.

Unless otherwise notifed the term "haloalkyl" refers to straight or branched alkyl derivatives in which at least one hydrogen is substituted by a halogen atom, preferably by a fluoro atom. For example, the term halopropyl comprises perfluoro- *ⁿ*-propyl and perfluoro*^{iso}*-propyl, as well as 1-chloro propyl, the term haloethyl comprises 1-fluoroethyl, 2,2,2- trifluoroethyl, 1-chloroethyl and 2-chloroethyl.

Unless otherwise notifed the term "alkoxy" refers to straight or branched derivatives. For example, the term propoxy comprises *ⁿ*-propoxy and *^{iso}*-propoxy, the term butoxy comrises *ⁿ*-butoxy, *^{iso}*-butoxy and *^{tert.}*-butoxy.

Compounds of general formula I, in which in which at least one of the groups R¹-R³ is selected from the group consisting of fluoro, chloro, hydroxy, C₁-C₃-alkyl, C₁-C₃-alkoxy, or in which two of the groups R¹-R³ together form a group -O-CH₂-O- or -CH₂-CH₂-O- are a preferred subject of the invention.

Preferred groups R⁴ are hydrogen and C₁-C₃-alkyl.

Preferred groups R⁵ are phenyl, fluorophenyl, fluoropyridinyl, methylphenyl, dimethylphenyl, difluorophenyl.

Preferred groups R⁶ are hydrogen, fluoro, chloro, C₁-C₃-alkyl, C₁-C₃-alkoxy, especially preferred are hydrogen and C₁-C₃-alkyl.

Preferred groups R⁷ and R⁸ are hydrogen and the other is methyl or ethyl or in which both of R⁷ and R⁸ are methyl or ethyl.One especially preferred embodiment are the compounds disclosed in the examples and the group of compounds built by the subcombination of all residues as disclosed in the compounds of the examples.

One aspect of the invention are the following compounds of formula I 5-Amino-1-(4-fluorophenyl)-*N*-[(*cis*)-6-fluoro-1,2,3,4-tetrahydro-2-hydroxy-5-methoxy-4,4-dimethyl-2-(trifluoromethyl)naphthalene-1-yl]-1*H*-pyrazole-4-carboxamide,
5-Amino-1-(4-fluorophenyl)-*N*-[(*cis*)-6-fluoro-1,2,3,4-tetrahydro-2,5-dihydroxy-4,4-dimethyl-2-(trifluoromethyl)naphthalene-1-yl]-1*H*-pyrazole-4-carboxamide,
5-Amino-1-(2,4-difluorophenyl)-*N*-[(*cis*)-6-fluoro-1,2,3,4-tetrahydro-2-hydroxy-5-methoxy-4,4-dimethyl-2-(trifluoromethyl)naphthalene-1-yl]-1*H*-pyrazole-4-carboxamide
5-Amino-1-(2,4-difluorophenyl)-*N*-[(*cis*)-6-fluoro-1,2,3,4-tetrahydro-2,5-dihydroxy-4,4-dimethyl-2-(trifluoromethyl)naphthalene-1-yl]-1*H*-pyrazole-4-carboxamide,
5-Amino-[6,7-difluoro-1,2,3,4-tetrahydro-2,5-dihydroxy-4-ethyl-2-(trifluoromethyl)naphthalen-1-yl]-1-(4-fluorophenyl)-1*H*-imidazole-4-carboxamide,
5-Amino-1-(6-fluoropyridin-3-yl)-*N*-[(*cis*)-6-fluoro-1,2,3,4-tetrahydro-2-hydroxy-5-methoxy-4,4-dimethyl-2-(trifluoromethyl)naphthalene-1-yl]-1*H*-pyrazole-4-carboxamide,
5-Amino-1-(6-fluoropyridin-3-yl)-*N*-[(*cis*)-6-fluoro-1,2,3,4-tetrahydro-2,5-dihydroxy-4,4-dimethyl-2-(trifluoromethyl)naphthalene-1-yl]-1*H*-pyrazole-4-carboxamide,
5-Amino-1-(4-fluorophenyl)- *N*-[(*1*□,*2*□,*4*□)-6-fluoro-1,2,3,4-tetrahydro-2,5-dihydroxy-4-ethyl-2-(trifluoromethyl)naphthalene-1-yl]-1*H*-pyrazole-4-carboxamide,
5-Amino-1-(6-fluoropyridin-3-yl)-*N*-[(*cis*)-6-fluoro-1,2,3,4-tetrahydro-2-hydroxy-5-methoxy-4,4-dimethyl-2-(trifluoromethyl)naphthalene-1-yl]-1*H*-pyrrole-4-carboxamide,
5-Amino-1-(6-fluoropyridin-3-yl)-*N*-[(*1*□,*2*□,*4*□)-6-fluoro-1,2,3,4-tetrahydro-2,5-dihydroxy-4-ethyl-2-(trifluoromethyl)naphthalene-1-yl]-1*H*-pyrrole-4-carboxamide,
5-Amino-1-(6-fluoropyridin-3-yl)-*N*-[(*1*□,*2*□,*4*□)-6-fluoro-1,2,3,4-tetrahydro-2,5-dihydroxy-4-ethyl-2-(trifluoromethyl)naphthalene-1-yl]-1*H*-pyrrole-4-carboxamide,
5-Amino-1-(2-fluoropyridin-4-yl)-*N*-[(*cis*)-6-fluoro-1,2,3,4-tetrahydro-2-hydroxy-5-methoxy-4,4-dimethyl-2-(trifluoromethyl)naphthalene-1-yl]-1*H*-pyrazole-4-carboxamide,
5-Amino-1-(4-fluorophenyl)-*N*-[(*cis*)-6-fluoro-1,2,3,4-tetrahydro-2,5-dihydroxy-3,4-dimethyl-2-(trifluoromethyl)naphthalene-1-yl]-1*H*-pyrazole-4-carboxamide,
5-Amino-1-(2,4-difluorophenyl)-*N*-[(*cis*)-6-fluoro-1,2,3,4-tetrahydro-2,5-dihydroxy-4,4-dimethyl-2-(trifluoromethyl)naphthalene-1-yl]-1*H*-pyrazole-4-carboxamide,
5-Amino-1-(4-fluorophenyl)-*N*-[(*cis*)-6-fluoro-1,2,3,4-tetrahydro-2,5-dihydroxy-3,4-dimethyl-2-(trifluoromethyl)naphthalene-1-yl]-1*H*-pyrrole-4-carboxamide,
5-Amino-[6,7-difluoro-1,2,3,4-tetrahydro-2,5-dihydroxy-4-ethyl-2-(trifluoromethyl)naphthalen-1-yl]-1-(4-fluorophenyl)-1*H*-pyrazole]-4-carboxamide,
5-Amino-1-(4-fluorophenyl)-*N*-[(*cis*)-6-fluoro-1,2,3,4-tetrahydro-2-hydroxy-5-methoxy-4,4-dimethyl-2-(trifluoromethyl)naphthalene-1-yl]-1*H*-imidazole-4-carboxamide,
5-5-Amino-1-(4-fl uorophenyl)-*N*-[(cis)-6-fluoro-1,2,3,4-tetrahydro-2,5-dihydroxy-4,4-dimethyl-2-(trifluoromethyl)naphthalene-1-yl]-1*H*-imidazole-4-carboxamide and
5-Amino-[6,7-difluoro-1,2,3,4-tetrahydro-2,5-dihydroxy-4-ethyl-2-(trifluoromethyl)naphthalen-1-yl]-1-(4-fluorophenyl)-1*H*-1,2,3-triazole-4-carboxamide.

Another aspect of the invention are the following compounds of formula I 5-Amino-1-(4-fluorophenyl)-*N*-[(*cis*)-6-fluoro-1,2,3,4-tetrahydro-2-hydroxy-5-methoxy-4,4-dimethyl-2-(trifluoromethyl)naphthalene-1-yl]-1*H*-pyrazole-4-carboxamide,
5-Amino-1-(4-fluorophenyl)-*N*-[(*cis*)-6-fluoro-1,2,3,4-tetrahydro-2,5-dihydroxy-4,4-dimethyl-2-(trifluoromethyl)naphthalene-1-yl]-1*H*-pyrazole-4-carboxamide,
5-Amino-1-(2,4-difluorophenyl)-*N*-[(*cis*)-6-fluoro-1,2,3,4-tetrahydro-2-hydroxy-5-methoxy-4,4-dimethyl-2-(trifluoromethyl)naphthalene-1-yl]-1*H*-pyrazole-4-carboxamide,
5-Amino-1-(2,4-difluorophenyl)-*N*-[(*cis*)-6-fluoro-1,2,3,4-tetrahydro-2,5-dihydroxy-4,4-dimethyl-2-(trifluoromethyl)naphthalene-1-yl]-1*H*-pyrazole-4-carboxamide and
5-Amino-[6,7-difluoro-1,2,3,4-tetrahydro-2,5-dihydroxy-4-ethyl-2-(trifluoromethyl)naphthalen-1-yl]-1-(4-fluorophenyl)-1*H*-imidazole-4-carboxamide.

In addition, the invention relates to the use of the compounds of general formula I for the production of pharmaceutical agents as well as their use for the production of pharmaceutical agents for treating inflammatory diseases.

The C₁-C₅-alkyl groups can be straight-chain or branched and stand for a methyl, ethyl, n-propyl, iso-propyl, n-butyl, iso-butyl, tert-butyl or n-pentyl group, or a 2,2-dimethylpropyl, 2-methylbutyl or 3-methylbutyl group. A methyl or ethyl group is preferred. They can optionally be substituted by 1-3 hydroxy, 1-3 C₁-C₅-alkoxy and/or 1-3 COOR⁶ groups. Preferred are hydroxy groups.

The C₁-C₅-alkoxy groups can be straight-chain or branched and stand for a methoxy, ethoxy, n-propoxy, iso-propoxy, n-butoxy, iso-butoxy, tert-butoxy or n-pentoxy, 2,2-dimethylpropoxy, 2-methylbutoxy or 3-methylbutoxy group. A methoxy or ethoxy group is preferred.

The designation halogen atom or halogen means a fluorine, chlorine, bromine or iodine atom. Preferred is a fluorine, chlorine or bromine atom. More preferred is a fluorine or chlorine atom. Most preferred is a fluorine atom.

Due to the presence of asymmetry centers, the compounds according to the invention of the general formula I occur as stereoisomers. All possible enantiomers, and if several asymmetric centres are present all possible diastereomers (e.g.: RR, RS, SR, SS), both in enantiomerically pure form and also as racemates, both as pure diastereomers and also as diastereomer mixtures, are objects of the present invention.

Prodrugs are defined as compounds that are cleaved into the compounds that are claimed by metabolism in the organism or by contact with the organism. The prodrugs are subject to at least one biotransformatory step until the claimed compounds are released, which then exert their pharmacological effect. Prodrugs of the presently claimed compounds are also an aspect of the invention. Examples of prodrugs are esters and amides built wherever chemically possible due to the individual residues at a specific compound of formula I as claimed.

The compounds according to the invention can also be present in the form of salts with physiologically compatible anions, for example in the form of hydrochlorides, sulfates, nitrates, phosphates, pivalates, maleates, fumarates, tartrates, benzoates, mesylates, citrates or succinates.

The process for the production of the compounds and intermediates of WO2005/034939, WO2006/027236, WO2006/066950, WO2006/100100 , WO2006/108712 , WO2006/108699 and WO2007/000334 can also be used for the production of the compounds and intermediates according to the invention.

An amino tetrahydronaphthaline of general formula (II), in which , R¹, R², R³, R⁶, R⁷ and R⁸ have the meanings that are indicated for formula (I), is coupled with an acid of the general formula (III), in which under X, Y, R⁴ and R⁵ have the meaning that is indicated for formula (I), under usage of amide coupling reagents known to the expert like HATU, TBTU or HBTU or via formation of the acid chloride to compounds of the general formula (I) according to the invention.

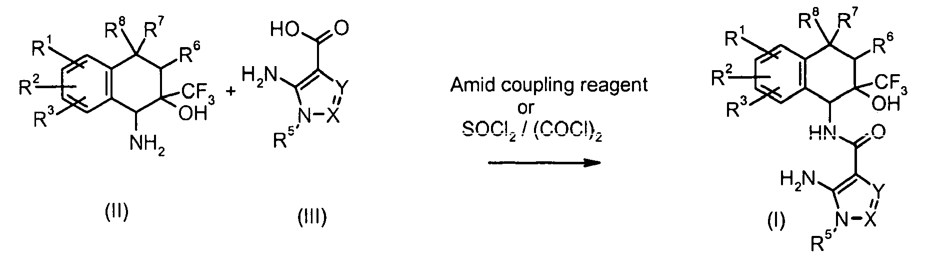

If the compounds according to the invention are present as racemic mixtures, they can be separated into pure, optically active forms according to the methods of racemate separation that are familiar to one skilled in the art. For example, the racemic mixtures can be separated by chromatography on an even optically active carrier material (CHIRALPAK AD^{®}) into the pure isomers. It is also possible to esterify the free hydroxy group in a racemic compound of general formula I with an optically active acid and to separate the diastereoisomeric esters that are obtained by fractionated crystallization or by chromatography, and to saponify the separated esters in each case to the optically pure isomers. As an optically active acid, for example, mandelic acid, camphorsulfonic acid or tartaric acid can be used.

The binding of the substances to the glucocorticoid receptor (GR) and other steroid hormone receptors (mineral corticoid receptor (MR), progesterone receptor (PR) and androgen receptor (AR)) is examined with the aid of recombinantly produced receptors. Cytosol preparations of Sf9 cells, which had been infected with recombinant baculoviruses, which code for the GR, are used for the binding studies. In comparison to reference substance [³H]-dexamethasone, the substances show a high to very high affinity to GR. Example 3 thus shows, e.g., the following profile: IC₅₀(GR) = 6 nmol; IC₅₀(MR), IC₅₀(PR), IC₅₀(AR), IC₅₀(ER) > 1 µmol.

As an essential, molecular mechanism for the anti-inflammatory action of glucocorticoids, the GR-mediated inhibition of the transcription of cytokines, adhesion molecules, enzymes and other pro-inflammatory factors is considered. This inhibition is produced by an interaction of the GR with other transcription factors, e.g., AP-1 and NF-kappa-B (for a survey, see Cato, A. C. B., and Wade, E., BioEssays 18, 371-378, 1996).

The compounds of general formula I according to the invention inhibit the secretion of cytokine IL-8 into the human monocyte cell line THP-1 that is triggered by lipopolysaccharide (LPS). The concentration of the cytokines was determined in the supernatant by means of commercially available ELISA kits. The compound of Example 3 showed an inhibition IC₅₀(IL8) = 7,1 nM (96 % eff. relative to dexamethasone).

The anti-inflammatory action of the compounds of general formula I was tested in the animal experiment by tests in the croton oil-induced inflammation in rats and mice (J. Exp. Med. (1995), **182**, 99-108). To this end, croton oil in ethanolic solution was applied topically to the animals' ears. The test substances were also applied topically or systemically at the same time or two hours before the croton oil. After 16-24 hours, the ear weight was measured as a yardstick for inflammatory edema, the peroxidase activity as a yardstick for the invasions of granulocytes, and the elastase activity as a yardstick for the invasion of neutrophilic granulocytes. In this test, the compounds of general formula I inhibit the three above-mentioned inflammation parameters both after topical administration and after systemic administration.

One of the most frequent undesirable actions of a glucocorticoid therapy is the so-called "steroid diabetes" [cf., Hatz, H. J., Glucocorticoide: Immunologische Grundlagen, Pharmakologie und Therapierichtlinien [Glucocorticoids: Immunological Bases, Pharmacology and Therapy Guidelines], Wissenschaftliche Verlagsgesellschaft mbH, Stuttgart, 1998]. The reason for this is the stimulation of gluconeogenesis in the liver by induction of the enzymes responsible in this respect and by free amino acids, which are produced from the degradation of proteins (catabolic action of glucocorticoids). A key enzyme of the catabolic metabolism in the liver is tyrosinamino transferase (TAT). The activity of this enzyme can be determined from liver homogenates by photometry and represents a good measurement of the undesirable metabolic actions of glucocorticoids. To measure the TAT induction, the animals are sacrificed 8 hours after the test substances are administered, the livers are removed, and the TAT activity is measured in the homogenate. In this test, at doses in which they have an anti-inflammatory action, the compounds of general formula I induce little or no tyrosinamino transferase.

Because of their anti-inflammatory and, in addition, anti-allergic, immunosuppressive and antiproliferative action, the compounds of general formula I according to the invention can be used as medications for treatment or prophylaxis of the following pathologic conditions in mammals and humans: in this case, the term "DISEASE" stands for the following indications:
(i) Lung diseases that are accompanied by inflammatory, allergic and/or proliferative processes:
   - Chronic, obstructive lung diseases of any origin, primarily bronchial asthma
   - Bronchitis of different origins
   - All forms of restrictive lung diseases, primarily allergic alveolitis,
   - All forms of pulmonary edema, primarily toxic pulmonary edema
   - Sarcoidoses and granulomatoses, especially Boeck's disease
(ii) Rheumatic diseases/autoimmune diseases/joint diseases that are accompanied by inflammatory, allergic and/or proliferative processes:
   - All forms of rheumatic diseases, especially rheumatoid arthritis, acute rheumatic fever, polymyalgia rheumatica
   - Reactive arthritis
   - Inflammatory soft-tissue diseases of other origins
   - Arthritic symptoms in the case of degenerative joint diseases (arthroses)
   - Traumatic arthritides
   - Collagenoses of any origin, e.g., systemic lupus erythematodes, sclerodermia, polymyositis, dermatomyositis, Sjögren's syndrome, Still's syndrome, Felty's syndrome
(iii) Allergies that are accompanied by inflammatory and/or proliferative processes:
   - All forms of allergic reactions, e.g., Quincke's edema, hay fever, insect bites, allergic reactions to pharmaceutical agents, blood derivatives, contrast media, etc., anaphylactic shock, urticaria, contact dermatitis
(iv) Vascular inflammations (vasculitides)
   - Panarteritis nodosa, temporal arteritis, erythema nodosum
(v) Dermatological diseases that are accompanied by inflammatory, allergic and/or proliferative processes:
   - Atopic dermatitis (primarily in children)
   - Psoriasis
   - Pityriasis rubra pilaris
   - Erythematous diseases, triggered by different noxae, e.g., radiation, chemicals, burns, etc.
   - Bullous dermatoses
   - Diseases of the lichenoid group,
   - Pruritis (e.g., of allergic origin)
   - Seborrheal eczema
   - Rosacea
   - Pemphigus vulgaris
   - Erythema exudativum multiforme
   - Balanitis
   - Vulvitis
   - Hair loss such as alopecia areata
   - Cutaneous T-cell lymphoma
(vi) Kidney diseases that are accompanied by inflammatory, allergic and/or proliferative processes:
   - Nephrotic syndrome
   - All nephritides
(vii) Liver diseases that are accompanied by inflammatory, allergic and/or proliferative processes:
   - Acute liver cell decomposition
   - Acute hepatitis of different origins, e.g., viral, toxic, pharmaceutical agent-induced
   - Chronic aggressive hepatitis and/or chronic intermittent hepatitis
(viii) Gastrointestinal diseases that are accompanied by inflammatory, allergic and/or proliferative processes:
   - Regional enteritis (Crohn's disease)
   - Colitis ulcerosa
   - Gastritis
   - Reflux esophagitis
   - Ulcerative colitis of other origins, e.g., native sprue
(ix) Proctologic diseases that are accompanied by inflammatory, allergic and/or proliferative processes:
   - Anal eczema
   - Fissures
   - Hemorrhoids
   - Idiopathic proctitis
(x) Eye diseases that are accompanied by inflammatory, allergic and/or proliferative processes:
   - Allergic keratitis, uveitis, iritis
   - Conjunctivitis
   - Blepharitis
   - Optic neuritis
   - Chorioiditis
   - Sympathetic ophthalmia
(xi) Diseases of the ear-nose-throat area that are accompanied by inflammatory, allergic and/or proliferative processes:
   - Allergic rhinitis, hay fever
   - Otitis externa, e.g., caused by contact dermatitis, infection, etc.
   - Otitis media
(xii) Neurological diseases that are accompanied by inflammatory, allergic and/or proliferative processes:
   - Cerebral edema, primarily tumor-induced cerebral edema
   - Multiple sclerosis
   - Acute encephalomyelitis
   - Meningitis
   - Various forms of convulsions, e.g., infantile nodding spasms
(xiii) Blood diseases that are accompanied by inflammatory, allergic and/or proliferative processes:
   - Acquired hemolytic anemia
   - Idiopathic thrombocytopenia
(xiv) Tumor diseases that are accompanied by inflammatory, allergic and/or proliferative processes:
   - Acute lymphatic leukemia
   - Malignant lymphoma
   - Lymphogranulomatoses
   - Lymphosarcoma
   - Extensive metastases, mainly in breast, bronchial and prostate cancers
(xv) Endocrine diseases that are accompanied by inflammatory, allergic and/or proliferative processes:
   - Endocrine orbitopathy
   - Thyreotoxic crisis
   - De Quervain's thyroiditis
   - Hashimoto's thyroiditis
   - Basedow's disease
(xvi) Organ and tissue transplants, graft-versus-host disease
(xvii) Severe shock conditions, e.g., anaphylactic shock, systemic inflammatory response syndrome (SIRS)
(xviii) Substitution therapy in:
   - Innate primary suprarenal insufficiency, e.g., congenital adrenogenital syndrome
   - Acquired primary suprarenal insufficiency, e.g., Addison's disease, autoimmune adrenalitis, meta-infective tumors, metastases, etc.
   - Innate secondary suprarenal insufficiency, e.g., congenital hypopituitarism
   - Acquired secondary suprarenal insufficiency, e.g., meta-infective tumors, etc.
(xix) Vomiting that is accompanied by inflammatory, allergic and/or proliferative processes:
   - e.g., in combination with a 5-HT3 antagonist in cytostatic-agent-induced vomiting
(xx) Pains of inflammatory origins, e.g., lumbago.

Moreover, the compounds of general formula I according to the invention can be used for treatment and prophylaxis of additional pathologic conditions that are not mentioned above, for which synthetic glucocorticoids are now used (see in this respect Hatz, H. J., Glucocorticoide: Immunologische Grundlagen, Pharmakologie und Therapierichtlinien, Wissenschaftliche Verlagsgesellschaft mbH, Stuttgart, 1998).

All previously mentioned indications (i) to (xx) are described in more detail in Hatz, H. J., Glucocorticoide: Immunologische Grundlagen, Pharmakologie und Therapierichtlinien, Wissenschaftliche Verlagsgesellschaft mbH, Stuttgart, 1998.

For the therapeutic actions in the above-mentioned pathologic conditions, the suitable dose varies and depends on, for example, the active strength of the compound of general formula I, the host, the type of administration, and the type and severity of the conditions that are to be treated, as well as the use as a prophylactic agent or therapeutic agent.

In addition, the invention provides:
(i) The use of one of the compounds of formula I according to the invention or mixture thereof for the production of a medication for treating a DISEASE;
(ii) A process for treating a DISEASE, said process comprises an administration of an amount of the compound according to the invention, in which the amount suppresses the disease and in which the amount of compound is given to a patient who requires such a medication;
(iii) A pharmaceutical composition for treating a DISEASE, said treatment comprises one of the compounds according to the invention or mixture thereof and at least one pharmaceutical adjuvant and/or vehicle.

In general, satisfactory results can be expected in animals when the daily doses comprise a range of 1 µg to 100,000 µg of the compound according to the invention per kg of body weight. In the case of larger mammals, for example the human, a recommended daily dose lies in the range of 1 µg to 100,000 µg per kg of body weight. Preferred is a dose of 10 to 30,000 µg per kg of body weight, and more preferred is a dose of 10 to 10,000 µg per kg of body weight. For example, this dose is suitably administered several times daily. For treating acute shock (e.g., anaphylactic shock), individual doses can be given that are significantly above the above-mentioned doses.

The formulation of the pharmaceutical preparations based on the new compounds is carried out in a way that is known in the art by the active ingredient being processed with the vehicles that are commonly used in galenicals, fillers, substances that influence decomposition, binding agents, moisturizers, lubricants, absorbents, diluents, flavoring correctives, coloring agents, etc., and converted into the desired form of administration. In this case, reference is made to Remington's Pharmaceutical Science, 15th Edition, Mack Publishing Company, East Pennsylvania (1980).

For oral administration, especially tablets, coated tablets, capsules, pills, powders, granulates, lozenges, suspensions, emulsions or solutions are suitable.

For parenteral administration, injection and infusion preparations are possible.

For intra-articular injection, correspondingly prepared crystal suspensions can be used.

For intramuscular injection, aqueous and oily injection solutions or suspensions and corresponding depot preparations can be used.

For rectal administration, the new compounds can be used in the form of suppositories, capsules, solutions (e.g., in the form of enemas) and ointments both for systemic and for local treatment.

For pulmonary administration of the new compounds, the latter can be used in the form of aerosols and inhalants.

For local application to eyes, outer ear channels, middle ears, nasal cavities, and paranasal sinuses, the new compounds can be used as drops, ointments and tinctures in corresponding pharmaceutical preparations.

For topical application, formulations in gels, ointments, fatty ointments, creams, pastes, powders, milk and tinctures are possible. The dosage of the compounds of general formula I should be 0.01%-20% in these preparations to achieve a sufficient pharmacological action.

The invention also comprises the compounds of general formula I according to the invention as therapeutic active ingredients.

In addition, the compounds of general formula I according to the invention are part of the invention as therapeutic active ingredients together with pharmaceutically compatible and acceptable adjuvants and vehicles.

The invention also comprises a pharmaceutical composition that contains one of the pharmaceutically active compounds according to the invention or mixtures thereof or a pharmaceutically compatible salt thereof and a pharmaceutically compatible salt or pharmaceutically compatible adjuvants and vehicles.

The compounds of general formula (I) according to the invention can optionally also be formulated and/or administered in combination with other active ingredients.

The invention therefore also relates to combination therapies or combined compositions, in which a compound of general formula (I) or a pharmaceutically acceptable salt thereof, or a pharmaceutical composition that contains a compound of general formula (I) or a pharmaceutically acceptable salt thereof, is administered either simultaneously (optionally in the same composition) or in succession together with one or more pharmaceutical agents for treating one of the above-mentioned pathologic conditions. For example, for treatment of rheumatoid arthritis, osteoarthritis, COPD (chronic obstructive lung disease), asthma or allergic rhinitis, a compound of general formula (I) of this invention can be combined with one or more pharmaceutical agents for treating such a condition. When such a combination is administered by inhalation, the pharmaceutical agent that is to be combined can be selected from the following list:
- A PDE4 inhibitor including an inhibitor of the PDE4D isoform,
- A selective β.sub2.adrenoceptor agonist, such as, for example, metaproterenol, isoproterenol, isoprenaline, albuterol, salbutamol, formoterol, salmeterol, terbutaline, orcipresnaline, bitolterol mesylate, pirbuterol or indacaterol;
- A muscarine receptor antagonist (for example, an M1, M2 or M3 antagonist, such as, for example, a more selective M3 antagonist), such as, for example, ipratropium bromide, tiotropium bromide, oxitropium bromide, pirenzepine or telenzepine;
- A modulator of the chemokine receptor function (such as, for example, a CCR1 receptor antagonist); or
- An inhibitor of the p38 kinase function.

For another subject of this invention, such a combination with a compound of general formula (I) or a pharmaceutically acceptable salt thereof is used for treatment of COPD, asthma or allergic rhinitis and can be administered by inhalation or orally in combination with xanthine (such as, for example, aminophylline or thyeophylline), which also can be administered by inhalation or orally.

### Experimental Part:

The various aspects of the invention described in this application are illustrated by the following examples which are not meant to limit the invention in any way.

### Example 1

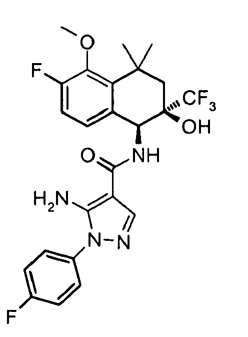

### 5-Amino-1-(4-fluorophenyl)-N-[(cis)-6-fluoro-1,2,3,4-tetrahvdro-2-hydroxy-5-methoxy-4,4-dimethyl-2-(trifluoromethyl)naphthalene-1-yl]-1H-pyrazole-4-carboxamide

31 mg (81 µmol) HATU are dissolved in 200 µl DMF. 28 µl diisopropyl ethyl amine and 18 mg (81 µM) 5-amino-1-(4-fluorophenyl)-1H-pyrazole-4-carboxylic acid are added and the mixture is shaken for 10 minutes. 25 mg (81 µM) (*cis*)-1-amino-1,2,3,4-tetrahydro-5-methoxy-4,4-dimethyl-2-(trifluoromethyl)naphthalene-2-ol (W02006/108712) are added and the mixture is shaken until the amine is solved. After 16 hours the mixture is diluted with trichloromethan and directly chromatographed on silica gel (2 x 20 cm x 20 cm TLC plates, hexane / ethyl acetate 50%). 31 mg of the desired product are obtained.
¹H-NMR (CDCl₃); δ = 1.49 (s, 3H), 1.65 (s, 3H), 2.03 (d, 1 H), 2.11 (d, 1 H), 3.96 (s, 3H), 5.38 (br., 1H), 5.54 (s, 2H), 6.45 (d, 1H), 6.96 (dd, 1H), 7.02 (dd, 1H), 7.23 (dd, 2H), 7.54 (dd, 2H), 7.79 (s, 1 H).

### Example 2

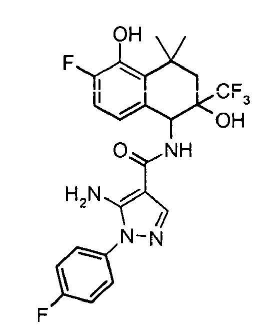

### 5-Amino-1-(4-fluorophenyl)-N-[(cis)-6-fluoro-1,2,3,4-tetrahvdro-2,5-dihvdroxy-4,4-dimethyl-2-(trifluoromethyl)naphthalene-1-yl]-1H-pyrazole-4-carboxamide

15 mg (28µmol) 5-Amino-1-(4-fluorophenyl)-*N*-[(*cis*)-6-fluoro-1,2,3,4-tetrahydro-2-hydroxy-5-methoxy-4,4-dimethyl-2-(trifluoromethyl)naphthalene-1yl]-1 *H*-pyrazole-4-carboxamide in 3 ml of CH₂Cl₂, are mixed at -30°C with 1,5 ml of 1 M boron tribromide solution in CH₂Cl₂. The mixture is warmed to 0°C over 5 hours and stirred for additional 16 hours while warming to room temperature. The batch is poured into saturated NaHCO₃ and ice, stirred for 10 minutes and extracted with ethyl acetate. The combined organic extracts are washed with brine, dried (Na₂SO₄) and concentrated by evaporation in a vacuum. After preparative thin layer chromatography on silica gel (hexane / 2-propanol 9 %), 8 mg of the desired product are obtained.
¹H-NMR (CD₃OD); δ = 1.57 (s, 3H), 1.71 (s, 3H), 2.02 (d, 1H), 2.14 (d, 1 H), 5.54 (br., 1H), 6.77 (dd, 1H), 6.97 (d, 1 H), 7.33 (dd, 2H), 7.62 (dd, 1 H), 7.94 (s, 1 H).

### Example 3

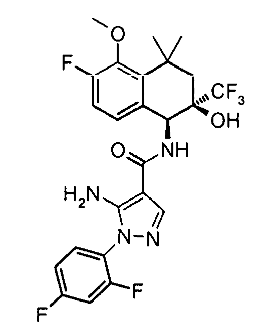

### 5-Amino-1-(2,4-difluorophenyl)-N-[(cis)-6-fluoro-1,2,3,4-tetrahydro-2-hydroxy-5-methoxy-4,4-dimethyl-2-(trifluoromethyl)naphthalene-1-yl]-1H-pyrazole-4-carboxamide

31 mg (81 µmol) HATU are dissolved in 200 µl DMF. 28 µl diisopropyl ethylamine and 20 mg (81 µM)-5-amino-1-(2,4-difluorophenyl)-1H-pyrazole-4-carboxylic acid are added and the mixture is shaken for 10 minutes. 25 mg (81 µM) (*cis*)-1-amino-1,2,3,4-tetrahydro-5-methoxy-4,4-dimethyl-2-(trifluoromethyl)naphthalene-2-ol are added and the mixture is shaken until the amine is solved. After 16 hours the mixture is diluted with trichloro methan and directly chromatographed on silica gel (2 x 20cm x 20cm TLC plates, hexane / ethylacetate 50%). 36 mg of the desired product are obtained.
¹H-NMR (CDCl₃); δ = 1.49 (s, 3H), 1.64 (s, 3H), 2.02 (d, 1 H), 2.10 (d, 1 H), 3.95 (s, 3H), 5.40 (br., 1H), 5.44 (s, 2H), 6.45 (d, 1 H), 6.97 (dd, 1H), 7.02 (dd, 1 H), 7.05 (m, 2H), 7.51 (ddd, 1H), 7.72 (s, 1 H).

### Example 4

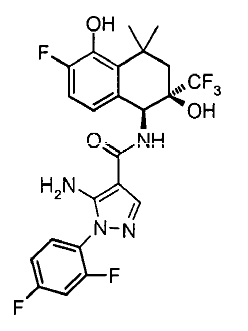

### 5-Amino-1-(2,4-difluorophenyl)-N-[(cis)-6-fluoro-1,2,3,4-tetrahydro-2,5-dihydroxy-4,4-dimethyl-2-(trifluoromethyl)naphthalene-1-yl]-1H-pyrazole-4-carboxamide

15 mg (28µmol) 5-Amino-1-(2,4-difluorophenyl)-*N*-[(*cis*)-6-fluoro-1,2,3,4-tetrahydro-2-hydroxy-5-methoxy-4,4-dimethyl-2-(trifluoromethyl)naphthalene-1-yl]-1*H*-pyrazole-4-carboxamide in 3 ml of CH₂Cl₂, are mixed at -30°C with 1,5 ml of 1 M boron tribromide solution in CH₂Cl₂. The mixture is warmed to 0°C over 5 hours and stirred for additional 16 hours while warming to room temperature. The batch is poured into saturated NaHCO₃ and ice, stirred for 10 minutes and extracted with ethyl acetate. The combined organic extracts are washed with brine, dried (Na₂SO₄) and concentrated by evaporation in a vacuum. After preparative thin layer chromatography on silica gel (hexane / 2-propanol 9 %), 10 mg of the desired product are obtained.
¹H-NMR (CD₃OD); δ = 1.57 (s, 3H), 1.71 (s, 3H), 2.03 (d, 1 H), 2.14 (d, 1 H), 5.53 (s, 1H), 6.78 (dd, 1 H), 6.98 (d, 1 H), 7.21 (dd, 1 H), 7.30 (dd, 1H),7.61 (ddd, 1H), 7.96 (s, 1 H).

### Example 5

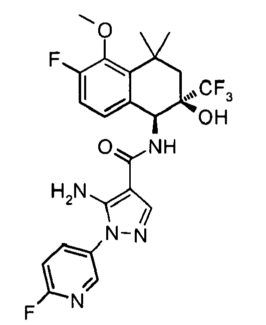

### 5-Amino-1-(6-fluoropyridin-3-yl)-N-[(cis)-6-fluoro-1,2,3,4-tetrahydro-2-hydroxy-5-methoxy-4,4-dimethyl-2-(trifluoromethyl)naphthalene-1-yl]-1H-pyrazole-4-carboxamide

Analogously to example 1 (*cis*)-1-amino-1,2,3,4-tetrahydro-5-methoxy-4,4-dimethyl-2-(trifluoromethyl)naphthalene-2-ol can be reacted with 5-amino-1-(6-fluoropyridin-3-yl)-1*H*-pyrazole-4-carboxylic acid using HATU as amid coupling agent.

### Example 6

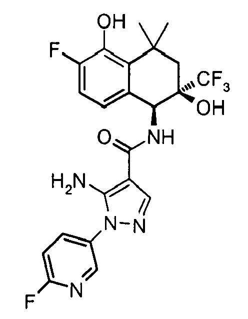

### 5-Amino-1-(6-fluoropyridin-3-yl)-N-[(cis)-6-fluoro-1,2,3,4-tetrahydro-2,5-dihydroxy-4,4-dimethyl-2-(trifluoromethyl)naphthalene-1-yl]-1H-pyrazole-4-carboxamide

Analogously to example 2 5-Amino-1-(6-fluoropyridin-3-yl)-N-[(cis)-6-fluoro-1,2,3,4-tetrahydro-2-hydroxy-5-methoxy-4,4-dimethyl-2-(trifluoromethyl)naphthalene-1-yl]-1*H*-pyrazole-4-carboxamide can be reacted with boron tribromide to give the ether cleaved product.

### Example 7

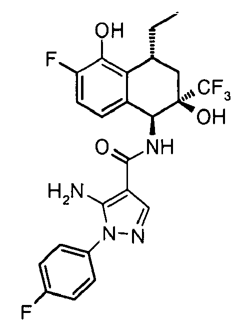

### 5-Amino-1-(4-fluorophenyl)-N-[(1α,2α,4β)-6-fluoro-1,2,3,4-tetrahydro-2,5-dihydroxy-4-ethyl-2-(trifluoromethyl)naphthalene-1-yl]-1H-pyrazole-4-carboxamide

Analogously to example 3 (*5*α,*6*α,*8*β)-5-amino-2-fluoro-5,6,7,8-tetrahydro-8-ethyl-6-(trifluoromethyl)naphthalene-1,6-diol can be reacted with 1-(4-fluorophenyl)-1*H-*pyrazole-4-carboxylic acid using HATU as the amid coupling reagent.

### Example 8

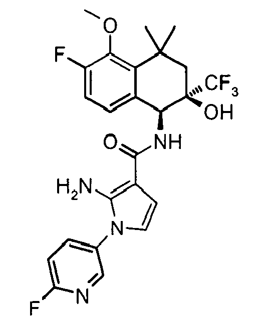

### 5-Amino-1-(6-fluoropyridin-3-yl)-N-[(cis)-6-fluoro-1,2,3,4-tetrahvdro-2-hydroxy-5-methoxy-4,4-dimethyl-2-(trifluoromethyl)naphthalene-1-yl]-1H-pyrrole-4-carboxamide

Analogously to example 3, 5-amino-2-fluoro-5,6,7,8-tetrahydro-8,8-dimethyl-6-(trifluoromethyl)naphthalene-1,6-diol can be reacted with 1-(6-fluoropyridin-3-yl)-1H-pyrrole-4-carboxylic acid using HATU as the amid coupling reagent.

### Example 9

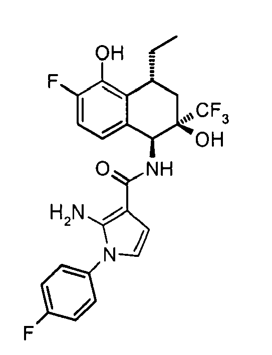

### 5-Amino-1-(6-fluoropvridin-3-yl)-N-[(1α,2α,4β)-6-fluoro-1,2,3,4-tetrahydro-2,5-dihydroxy-4-ethyl-2-(trifluoromethyl)naphthalene-1-yl]-1H-pyrrole-4-carboxamide

Analogously to example 3 (*5α*,*6α*,*8β*)-5-amino-2-fluoro-5,6,7,8-tetrahydro-8-ethyl-6-(trifluoromethyl)naphthalene-1,6-diol can be reacted with 1-(4-fluorophenyl)-1*H-*pyrrole-4-carboxylic acid using HATU as the amid coupling reagent.

### Example 10

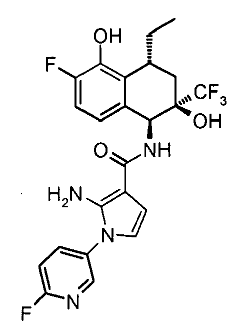

### 5-Amino-1-(6-fluoropyridin-3-yl)-N-[(1α,2α,4β)-6-fluoro-1,2,3,4-tetrahydro-2,5-dihydroxy-4-ethyl-2-(trifluoromethyl)naphthalene-1-yl]-1H-pyrrole-4-carboxamide

Analogously to example 3 (*5α*,*6α*,*8β*)-5-amino-2-fluoro-5,6,7,8-tetrahydro-8-ethyl-6-(trifluoromethyl)naphthalene-1,6-diol can be reacted with 1-(6-fluoropyridin-3-yl)-1*H-*pyrrole-4-carboxylic acid using HATU as the amid coupling reagent.

### Example 11

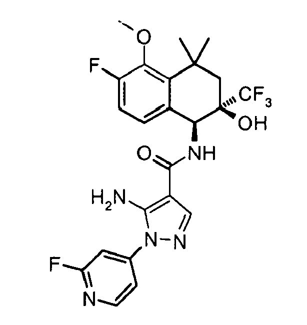

### 5-Amino-1-(2-fluoropyridin-4-yl)-N-[(cis)-6-fluoro-1,2,3,4-tetrahydro-2-hydroxy-5-methoxy-4,4-dimethyl-2-(trifluoromethyl)naphthalene-1-yl]-1H-pyrazole-4-carboxamide

Analogously to example 1 (*cis*)-1-amino-1,2,3,4-tetrahydro-5-methoxy-4,4-dimethyl-2-(trifluoromethyl)naphthalene-2-ol can be reacted with 5-amino-1-(2-fluoropyridin-4-yl)-1*H*-pyrazole-4-carboxylic acid using HATU as amid coupling agent.

### Example 12

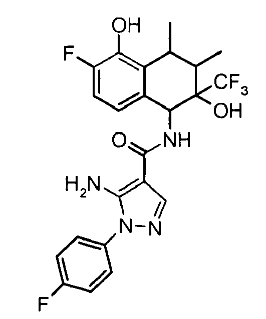

### 5-Amino-1-(4-fluorophenyl)-N-[(cis)-6-fluoro-1,2,3,4-tetrahydro-2,5-dihydroxy-3,4-dimethyl-2-(trifluoromethyl)naphthalene-1-yl]-1H-pyrazole-4-carboxamide Analogously to example 3 5-amino-2-fluoro-5,6,7,8-tetrahydro-7,8-dimethyl-6-(trifluoromethyl)naphthalene-1,6-diol can be reacted with 1-(4-fluorophenyl)-1H-pyrazole-4-carboxylic acid using HATU as the amid coupling reagent.

### Example 13

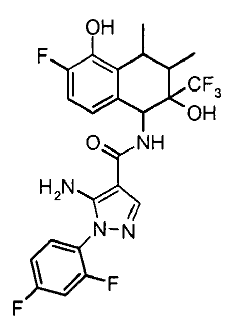

### 5-Amino-1-(2,4-difluorophenyl)-N-[(cis)-6-fluoro-1,2,3,4-tetrahydro-2,5-dihydroxy-4,4-dimethyl-2-(trifluoromethyl)naphthalene-1-yl]-1H-pyrazole-4-carboxamide

Analogously to example 3 5-amino-2-fluoro-5,6,7,8-tetrahydro-7,8-dimethyl-6-(trifluoromethyl)naphthalene-1,6-diol can be reacted with 1-(2,4-difluorophenyl)-1*H-*pyrazole-4-carboxylic acid using HATU as the amid coupling reagent.

### Example 14

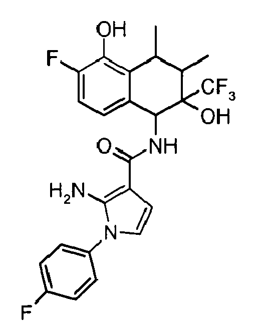

### 5-Amino-1-(4-fluorophenyl)-N-[(cis)-6-fluoro-1,2,3,4-tetrahydro-2,5-dihydroxy-3,4-dimethyl-2-(trifluoromethyl)naphthalene-1-yl]-1H-pyrrole-4-carboxamide

Analogously to example 3 5-amino-2-fluoro-5,6,7,8-tetrahydro-7,8-dimethyl-6-(trifluoromethyl)naphthalene-1,6-diol can be reacted with 1-(4-fluorophenyl)-1H-pyrrole-4-carboxylic acid using HATU as the amid coupling reagent.

### Example 15

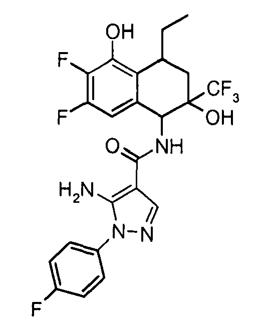

### 5-Amino-[6,7-difluoro-1,2,3,4-tetrahydro-2,5-dihydroxy-4-ethyl-2-(trifluoromethyl)naphthalen-1-yl]-1-(4-fluorophenyl)-1H-pyrazolel-4-carboxamide

Analogously to example 3 5-amino-2,3-difluoro-5,6,7,8-tetrahydro-8-ethyl-6-(trifluoromethyl)naphthalene-1,6-diol can be reacted with 1-(4-fluorophenyl)-1*H-*pyrazole-4-carboxylic acid using HATU as the amid coupling reagent.

### Example 16

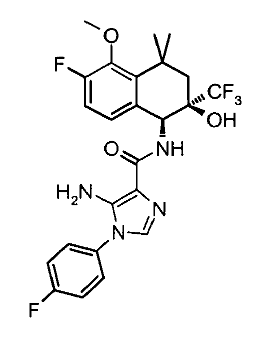

### 5-Amino-1-(4-fluorophenyl)-N-[(cis)-6-fluoro-1,2,3,4-tetrahydro-2-hydroxy-5-methoxy-4 4-dimethyl-2-(trifluoromethyl)naphthalene-1-yl]-1H-imidazole-4-carboxamide

### 1-(4-fluorophenyl)-1H-imidazole-4-carboxylic acid

A solution of amino-cyano-acetic acid ethyl ester (Ahn et al. J.Med.Chem, 1997, 40, p.2208) and triethyl orthoformate in 25 ml acetonitrile is heated under reflux for 45 minutes. After cooling to room temperature 1.27 ml (13.3 mmol) 4-fluoroaniline are added and the mixture is stirred for 16 hours. 400 mg 1-(4-fluorophenyl)-1H-imidazole-4-carboxylic acid ethyl ester precipitate out and are isolated by filtration. 1.5 g (6 mmol) 1-(4-fluorophenyl)-1H-imidazole-4-carboxylic acid ethyl ester in 7.5 ml ethanol and 30 ml NaOH are heated to 75°C for 3 hours. The pH of the solution is adjusted to pH 7 by 1 N HCl solution and the precipitating acid is collected by filtration. 922 mg of the desired acid are obtained after drying in vacuum. Analogously to example 3 5-amino-2-fluoro-5,6,7,8-tetrahydro-8-ethyl-6-(trifluoromethyl)naphthalene-1,6-ol can be reacted with 1-(4-fluorophenyl)-1H-imidazole-4-carboxylic acid using HATU as the amid coupling reagent.
¹H-NMR (CDCl₃); δ = 1.47 (s, 3H), 1.63 (s, 3H), 1.98 (d, 1 H), 2.13 (d, 1H), 5.00 (s, 1H), 5.21 (br, 1H), 6.91 (dd, 1 H), 7.03 (dd, 1 H), 7.13 (s, 1 H), 7.26 (dd, 2H), 7.41 (dd, 2H), 7.46 (d, 1 H).

### Example 17

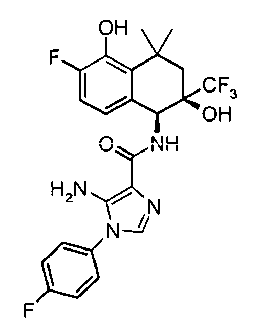

### 5-5-Amino-1-(4-fluorophenyl)-N-[(cis)-6-fluoro-1,2,3,4-tetrahydro-2,5-dihydroxy-4,4-dimethyl-2-(trifluoromethyl)naphthalene-1-yl]-1H-imidazole-4-carboxamide

Analogously to example 2 5-Amino-1-(4-fluorophenyl)-N-[(cis)-6-fluoro-1,2,3,4-tetrahydro-2-hydroxy-5-methoxy-4,4-dimethyl-2-(trifluoromethyl)naphthalene-1-yl]-1H-imidazole-4-carboxamide can be reacted with boron tribromide to give the ether cleaved product.

### Example 18

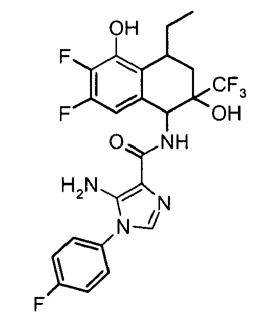

### 5-Amino-[6,7-difluoro-1,2,3,4-tetrahydro-2,5-dihydroxy-4-ethyl-2-(trifluoromethyl)naphthalen-1-yl]-1-(4-fluorophenyl)-1H-imidazole-4-carboxamide

Analogously to example 3 5-amino-2,3-difluoro-5,6,7,8-tetrahydro-8-ethyl-6-(trifluoromethyl)naphthalene-1,6-diol can be reacted with 1-(4-fluorophenyl)-1H-imidazole-4-carboxylic acid using HATU as the amid coupling reagent.

### Example 19

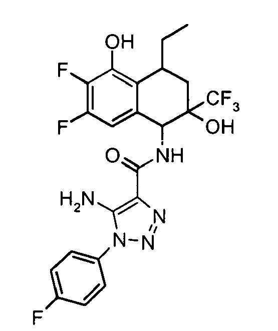

### 5-Amino-[6,7-difluoro-1,2,3,4-tetrahydro-2,5-dihydroxy-4-ethyl-2-(trifluoromethyl)naphthalen-1-yl]-1-(4-fluorophenyl)-1H-1,2,3-triazole-4-carboxamide

Analogously to example 3 5-amino-2,3-difluoro-5,6,7,8-tetrahydro-8-ethyl-6-(trifluoromethyl)naphthalene-1,6-diol can be reacted with 1-(4-fluorophenyl)-1H-triazole-4-carboxylic acid using HATU as the amid coupling reagent.

## Claims

1. Stereoisomers of general formula I 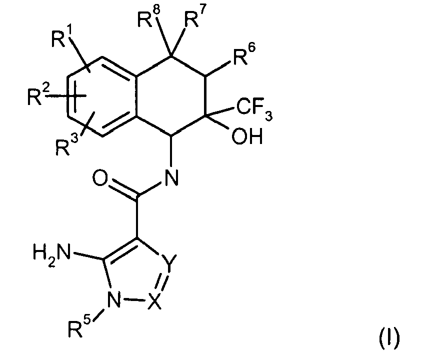 in which
X is N or C-R⁴
Y is N or C-R⁴
R¹, R², R³ independently of one another, are selected from the group consisting of hydrogen, halogen, cyano, nitro, hydroxy, or (C₁-C₅)-alkyl, (C₁-C₅)-halo-alkyl, (C₁-C₅)alkoxy, (C₁-C₅)halo-alkoxy and COOR⁹,
and in which two vicinal substituents together may form a group that is selected from the groups
-O-(CH₂)ₚ-O-, -O-(CH₂)ₚ-CH₂-, -O-CH=CH-, -(CH₂)ₚ₊₂-, -NH-(CH₂)ₚ₊₁-,
-N(C₁-C₃-alkyl)-(CH₂)ₚ₊₁-, and -NH-N=CH-,
in which p = 1 or 2, and in which R⁹ means hydrogen or C₁-C₄-alkyl
R⁴ is selected from the group consisting of hydrogen, (C₁-C₅)-alkyl, or (C₁-C₅)-halo-alkyl, and should two R⁴ moities be present in one molecule these have independent meanings,
R⁵ means a phenyl, pyridinyl or pyrimidinyl rest
which may have 1-3 substituents independently selected from the group consisting of halogen, cyano, nitro hydroxy, or (C₁-C₅)-alkyl, (C₁-C₅)-halo-alkyl, (C₁-C₅)alkoxy, (C₁-C₅)halo-alkoxy and COOR⁹, in which R⁹ has the above identified meaning,
and in which two vicinal substituents together may form a group that is selected from the groups
-O-(CH₂)ₚ-O-, -O-(CH₂)ₚ-CH₂-, -O-CH=CH-, -(CH₂)ₚ₊₂-, -NH-(CH₂)ₚ₊₁-,
-N(C₁-C₃-alkyl)-(CH₂)ₚ₊₁-, and -NH-N=CH-,
R⁶ is selected from the group consisting of hydrogen or (C₁-C₅)-alkyl or (C₁-C₅)-halo-alkyl
R⁷, R⁸ independently of one another, are selected from the group consisting of hydrogen or (C₁-C₅)-alkyl, (C₁-C₅)-halo-alkyl
or in which R⁶ and R⁷ together or R⁶ and R⁸ together or R⁷ and R⁸ together may form a C₃-C₈ cycloalkyl ring.

2. Stereoisomers according to claim 1, in which at least one of the groups R¹-R³ is selected from the group consisting of fluoro, chloro, hydroxy, C₁-C₃-alkyl, C₁-C₃-alkoxy,
or in which two of the groups R¹-R³ together form a group -O-CH₂-O- or -CH₂-CH₂-O-.

3. Stereoisomers according to claim 1, in R⁴ is selected from the group consisting of hydrogen, C₁-C₃-alkyl.

4. Stereoisomers according to claim 1, in which R⁵ is selected from the group consisting of phenyl, fluorophenyl, fluoropyridinyl, methylphenyl, dimethylphenyl, difluorophenyl.

5. Stereoisomers according to claim 1, in R⁶ is selected from the group consisting of hydrogen, C₁-C₃-alkyl.

6. Stereoisomers according to claim 1, in which one of R⁷ and R⁸ is hydrogen and the other is methyl or ethyl or
in which both of R⁷ and R⁸ are methyl or ethyl.

7. Use of the stereoisomers according to at least one of claims 1-6 for the manufacture of pharmaceutical agents.

8. Use of the stereoisomers according to at least one of claims 1-6 for the manufacture of pharmaceutical agents for treating inflammatory diseases.
